# EUROPEAN PATENT SPECIFICATION

(11) **EP 3 463 104 B1**
(45) Date of publication and mention of the grant of the patent: **09.10.2024**
(21) Application number: 17807421.7
(22) Date of filing: 31.05.2017
(51) Int. Cl.: A61B 10/04

(54) **PLIANT BIOPSY NEEDLE SYSTEM**
BIEGSAMES BIOPSIENADELSYSTEM
SYSTÈME D'AIGUILLE DE BIOPSIE FLEXIBLE

(30) Priority: 31.05.2016 US 201662343596 P
(43) Date of publication of application: 10.04.2019
(60) Divisional application: 24185724.2
(73) Proprietor: Intuitive Surgical Operations, Inc., Sunnyvale, CA 94086 (US)
(72) Inventor: GORDON, Lucas S., Sunnyvale, California 94086 (US); VALENCIA, Hans F., Sunnyvale, California 94086 (US); WAGNER, Oliver J., Sunnyvale, California 94086 (US)
(74) Representative: J A Kemp LLP
(86) International application number: PCT/US2017/035268
(87) International publication number: WO 2017/210333

(56) References cited:
- EP-A2- 0 966 920
- WO-A1-2007/067255
- WO-A1-2015/089372
- WO-A1-2015/089372
- CN-A- 104 971 406
- JP-A- H10 165 507
- US-A1- 2011 066 074
- US-A1- 2012 116 247
- US-A1- 2013 204 124
- US-A1- 2014 276 051
- US-A1- 2015 342 580
- US-A1- 2016 066 897

## Description

### FIELD

The present disclosure is directed to systems and methods for delivering a pliant biopsy needle through anatomical passageways.

### BACKGROUND

Minimally invasive medical techniques are intended to reduce the amount of tissue that is damaged during medical procedures, thereby reducing patient recovery time, discomfort, and harmful side effects. Such minimally invasive techniques may be performed through natural orifices in a patient anatomy or through one or more surgical incisions. Through these natural orifices or incisions clinicians may insert minimally invasive medical instruments (including surgical, diagnostic, therapeutic, or biopsy instruments) to reach a target tissue location. One such minimally invasive technique is to use a flexible and/or steerable elongate device, such as a flexible catheter that can be inserted into anatomic passageways and navigated toward a region of interest within the patient anatomy. Medical tools, such as biopsy instruments, are deployed through the catheter to perform a medical procedure at the region of interest. Medical tools are needed that are flexible enough to navigate the tight bends though the anatomic passageways while providing sufficient rigidity to ensure a predictable performance direction when deployed from the catheter.

US 2014/276051 A1 discloses a device for minimally invasive delivery of treatment substance. Systems, methods, and devices for delivering ablation fluid to a location of interest within a body (e.g., a location with the lung) are described. Ablation fluid delivery systems and devices can include flexible delivery needles within which a plurality of smaller flexible needles is housed. The smaller flexible needles can be configured to articulate or bend with respect to the flexible delivery needle to increase an effective delivery area for the ablation fluid. The smaller flexible needles can include apertures or other openings to facilitate delivery of the ablation fluid to the location of interest.

US 2012/116247 A1 discloses a bone marrow harvesting device including a flexible bone marrow harvesting needle that can bend during operation to prevent the needle tip from piercing the inner cortical wall of the target bone. The needle defines an aspiration channel that defines an intake end that is recessed to reduce the instances that the aspiration channel will be fouled by bone particles or other debris within the cancellous portion.

US 2015/342580 A1 discloses needles including elongate tubular cannulas having proximal portions, distal portions, and cannula walls defining cannula lumens. A distal portion of an elongate tubular cannula may include a distal end, one or more apertures disposed through, and along a first length of, the cannula wall in a pattern enhancing flexibility relative to a second length of the cannula wall lacking the apertures, and a sealing member disposed on the apertures. The apertures impart enhanced flexibility to the needle for navigating through tortuous pathways.

WO 2015/089372 A1 discloses a minimally invasive system comprising an elongate sheath and an elongate instrument slidably disposed within a lumen of the sheath. The sheath includes a flexible tube portion including a plurality of slots, a sheath element, and a rigid tube section, wherein the flexible tube portion is fixedly coupled to a distal end of the sheath element, and the rigid tube section is fixedly coupled to a distal end of the flexible tube. The lumen extends through the sheath element, the flexible tube portion, and the rigid tube section and defines a longitudinal axis of the sheath. The instrument includes a rigid distal portion adapted to slide between a retracted configuration in which the rigid distal portion is retracted within the rigid tube section and an extended configuration in which the rigid distal portion at least partially extends from the rigid tube section.

WO 2007/067255 A1 discloses an endoscopic instrument which comprises a first flexible insertion member sized for insertion through a body lumen to a target site and a needle coupled to the insertion member for penetration of tissue, the needle including a plurality of flexibility enhancing grooves formed therein along at least a first portion of the length of the needle. The preamble of claim 1 is based on this document.

JP H10 165507 A discloses an appliance for coronary artery dilation by PTCA, in which, when the guiding catheter is engaged with the coronary inlet part of the coronary artery and the appliance for dilation of the coronary artery is made to arrive at the deepest part of the coronary artery by the operation at the operator side, a cut groove of a suitable length heading toward the circumferential direction in the desired length range of an inside cylinder from the front end to the position near the coronary inlet part in the coronary artery or the mid-way position in the guiding catheter imparts a suitable elastic function to the inside cylinder, thereby preventing the increase in the pass resistance.

### SUMMARY

According to the present invention there is provided the medical tool of claim 1. Additional aspects of the invention are set out in the dependent claims,

The following description sets forth specific details describing some embodiments consistent with the present disclosure.

Consistent with some embodiments, a medical tool comprises an elongated tubular section having a body wall including a plurality of slits and a rigid needle tip coupled to a distal end of the tubular section. The tool further includes a flexible (e.g., polymer) jacket coupled with the elongated tubular section by extending into the plurality of slits which can, in some embodiments, block a fluid passageway through the plurality of slits.

Consistent with some non-claimed examples, a medical instrument system comprises a biopsy instrument including an elongated tubular section having a body wall including a plurality of slits, a rigid needle tip coupled to a distal end of the tubular section, and a flexible (e.g., polymer) jacket coupled to the elongated tubular section by extending into the plurality of slits which can, in some embodiments, block a fluid passageway through the plurality of slits. The instrument system also includes a sheath having a sheath channel sized to receive the biopsy instrument and includes a stylet formed of a super elastic material and sized to extend through the elongated tubular section and the rigid needle tip to straighten the elongated tubular section.

Consistent with some non-claimed examples, , a method comprises inserting a sheathed needle through a catheter and inserting a stylet through the needle. The stylet includes a superelastic material. The method also includes puncturing tissue with the needle and stylet; removing the stylet from the needle; applying a vacuum to the needle to collect a portion of the tissue inside the needle; and removing the needle and sheath from the catheter.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTIONS OF THE DRAWINGS

FIG. 1 is a simplified diagram of a teleoperated medical system according to some non-claimed examples.
FIG. 2A is a simplified diagram of a medical instrument system according to some non-claimed examples.
FIG. 2B is a simplified diagram of a medical instrument with an extended medical tool according to some non-claimed examples.
FIGS. 3A and 3B are simplified diagrams of side views of a patient coordinate space including a medical instrument mounted on an insertion assembly according to some non-claimed examples.
FIG. 4 is a perspective view of a medical tool according to some embodiments.
FIG. 5A is a transparent side view of a distal end of a biopsy tool according to some embodiments.
FIG. 5B is a transparent side view of the distal end of the biopsy tool of FIG. 4A retracted into a sheath.
FIG. 5C is an end view of the tool shaft with surface discontinuities.
FIG. 6 is a cross-sectional view of a distal end of a sheath according to some embodiments.
FIG. 7A is a side view of a distal end of a biopsy tool with a slit pattern according to one embodiment.
FIG. 7B is a side view of a distal end of a biopsy tool with a slit pattern according to another embodiment.
FIG. 8 is a side view of a distal end of a biopsy tool with at centered needle point according to another embodiment.
FIG.9 is a perspective view of a proximal end of a medical tool according to some embodiments.
FIG. 10A is a cross-sectional view of a proximal end of the medical tool of FIG. 8.
FIG. 10B is a bottom view of the hollow shaft.
FIG. 11 is a flowchart illustrating a method for using a medical instrument system according to some embodiments.
FIGS. 12A and 12B are perspective views of a proximal end of a medical tool according to some embodiments. FIG. 12B illustrates a retracted configuration of the medical instrument, and FIG. 12A illustrates an expanded configuration of the medical instrument.
FIG. 13 illustrates the coupling of medical tools to a medical instrument system of a teleoperated medical system according to some non-claimed examples.
FIG. 14 illustrates a cross-sectional view of a connector assembly according to some non-claimed examples.
FIG. 15A illustrates a connector key of the connector assembly of FIG. 14.
FIG. 15B illustrates the connector assembly of FIG. 14 in an uncompressed state.
FIG. 15C illustrates the connector assembly of FIG. 14 in a compressed state.
FIG. 16 illustrates a cylindrical section of a biopsy tool.
FIG. 17 illustrates a formerly cylindrical section of a biopsy tool cut longitudinally and unrolled into a planar form according to some embodiments.
FIG. 18 illustrates a formerly cylindrical section of a biopsy tool cut longitudinally and unrolled into a planar form according to some embodiments.
FIG. 19 illustrates an enlarged section of the unrolled section of FIG. 17.
FIGS. 20a and 20b illustrate side and top views, respectively, of a rigid distal tip of a biopsy tool according to some embodiments.
FIGS. 21 and 22 illustrate a distal end of a sheath according to some embodiments.

Embodiments of the present disclosure and their advantages are best understood by referring to the detailed description that follows. It should be appreciated that like reference numerals are used to identify like elements illustrated in one or more of the figures, wherein showings therein are for purposes of illustrating embodiments of the present disclosure and not for purposes of limiting the same.

### DETAILED DESCRIPTION

In the following description, specific details are set forth describing some embodiments consistent with the present disclosure. Numerous specific details are set forth in order to provide a thorough understanding of the embodiments. It will be apparent, however, to one skilled in the art that some embodiments may be practiced without some or all of these specific details. The specific embodiments disclosed herein are meant to be illustrative but not limiting. One skilled in the art may realize other elements that, although not specifically described here, are within the scope of the claims. In addition, to avoid unnecessary repetition, one or more features shown and described in association with one embodiment may be incorporated into other embodiments unless specifically described otherwise or if the one or more features would make an embodiment non-functional.

In some instances well known methods, procedures, components, and circuits have not been described in detail so as not to unnecessarily obscure aspects of the embodiments.

This disclosure describes various instruments and portions of instruments in terms of their state in three-dimensional space. As used herein, the term "position" refers to the location of an object or a portion of an object in a three-dimensional space (e.g., three degrees of translational freedom along Cartesian x-, y-, and z-coordinates). As used herein, the term "orientation" refers to the rotational placement of an object or a portion of an object (three degrees of rotational freedom - e.g., roll, pitch, and yaw). As used herein, the term "pose" refers to the position of an object or a portion of an object in at least one degree of translational freedom and to the orientation of that object or portion of the object in at least one degree of rotational freedom (up to six total degrees of freedom). As used herein, the term "shape" refers to a set of poses, positions, or orientations measured along an object.

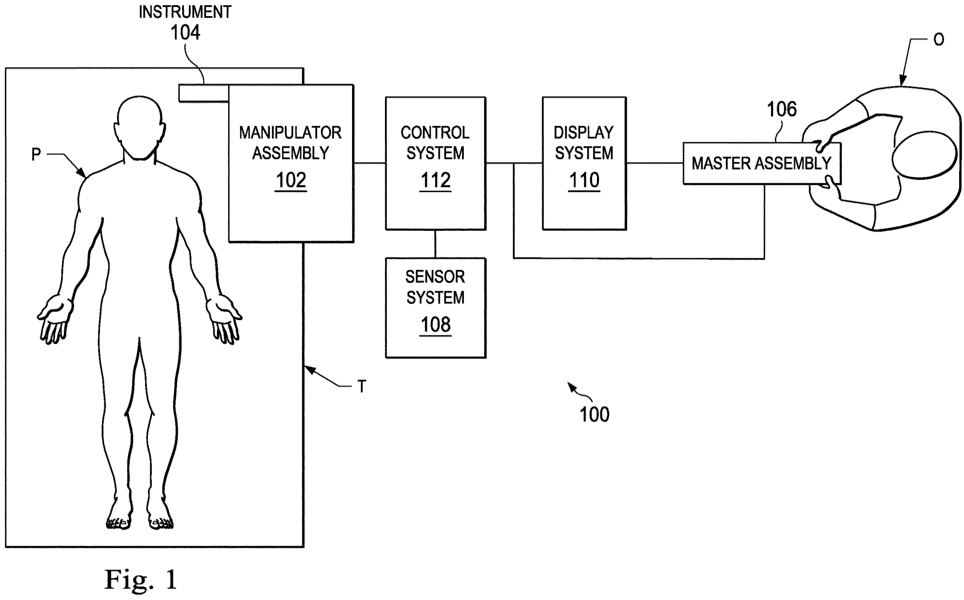

FIG. 1 is a simplified diagram of a teleoperated medical system 100 according to some non-claimed examples. In some embodiments, teleoperated medical system 100 may be suitable for use in, for example, surgical, diagnostic, therapeutic, or biopsy procedures. As shown in FIG. 1, medical system 100 generally includes a teleoperational manipulator assembly 102 for operating a medical instrument 104 in performing various procedures on a patient P. Teleoperational manipulator assembly 102 is mounted to or near an operating table T. A master assembly 106 allows an operator (e.g., a surgeon, clinician, or a physician O as illustrated in FIG. 1) to view the interventional site and to control teleoperational manipulator assembly 102.

Master assembly 106 may be located at a physician's console which is usually located in the same room as operating table T, such as at the side of a surgical table on which patient P is located. However, it should be understood that physician O can be located in a different room or a completely different building from patient P. Master assembly 106 generally includes one or more control devices for controlling teleoperational manipulator assembly 102. The control devices may include any number of a variety of input devices, such as joysticks, trackballs, data gloves, trigger-guns, hand-operated controllers, voice recognition devices, body motion or presence sensors, and/or the like. To provide physician O a strong sense of directly controlling instruments 104 the control devices may be provided with the same degrees of freedom as the associated medical instrument 104. In this manner, the control devices provide physician O with telepresence or the perception that the control devices are integral with medical instruments 104.

In some examples, the control devices may have more or fewer degrees of freedom than the associated medical instrument 104 and still provide physician O with telepresence. In some examples, the control devices may optionally be manual input devices which move with six degrees of freedom, and which may also include an actuatable handle for actuating instruments (for example, for closing grasping jaws, applying an electrical potential to an electrode, delivering a medicinal treatment, and/or the like).

Teleoperational manipulator assembly 102 supports medical instrument 104 and may include a kinematic structure of one or more non-servo controlled links (e.g., one or more links that may be manually positioned and locked in place, generally referred to as a set-up structure) and a teleoperational manipulator. Teleoperational manipulator assembly 102 may optionally include a plurality of actuators or motors that drive inputs on medical instrument 104 in response to commands from the control system (e.g., a control system 112). The actuators may optionally include drive systems that when coupled to medical instrument 104 may advance medical instrument 104 into a naturally or surgically created anatomic orifice. Other drive systems may move the distal end of medical instrument 104 in multiple degrees of freedom, which may include three degrees of linear motion (e.g., linear motion along the X, Y, Z Cartesian axes) and in three degrees of rotational motion (e.g., rotation about the X, Y, Z Cartesian axes). Additionally, the actuators can be used to actuate an articulable end effector of medical instrument 104 for grasping tissue in the jaws of a biopsy device and/or the like. Actuator position sensors such as resolvers, encoders, potentiometers, and other mechanisms may provide sensor data to medical system 100 describing the rotation and orientation of the motor shafts.

This position sensor data may be used to determine motion of the objects manipulated by the actuators.

Teleoperated medical system 100 may include a sensor system 108 with one or more sub-systems for receiving information about the instruments of teleoperational manipulator assembly 102. Such sub-systems may include a position/location sensor system (e.g., an electromagnetic (EM) sensor system); a shape sensor system for determining the position, orientation, speed, velocity, pose, and/or shape of a distal end and/or of one or more segments along a flexible body that may make up medical instrument 104; and/or a visualization system for capturing images from the distal end of medical instrument 104.

Teleoperated medical system 100 also includes a display system 110 for displaying an image or representation of the surgical site and medical instrument 104 generated by sub-systems of sensor system 108. Display system 110 and master assembly 106 may be oriented so physician O can control medical instrument 104 and master assembly 106 with the perception of telepresence.

In some examples, medical instrument 104 may have a visualization system (discussed in more detail below), which may include a viewing scope assembly that records a concurrent or real-time image of a surgical site and provides the image to the operator or physician O through one or more displays of medical system 100, such as one or more displays of display system 110. The concurrent image may be, for example, a two or three dimensional image captured by an endoscope positioned within the surgical site. In some examples, the visualization system includes endoscopic components that may be integrally or removably coupled to medical instrument 104. However in some examples, a separate endoscope, attached to a separate manipulator assembly may be used with medical instrument 104 to image the surgical site. In some examples, the endoscope may include one or more mechanisms for cleaning one or more lenses of the endoscope when the one or more lenses become partially and/or fully obscured by fluids and/or other materials encountered by the endoscope. In some examples, the one or more cleaning mechanisms may optionally include an air and/or other gas delivery system that is usable to emit a puff of air and/or other gassed to blow the one or more lenses clean. Examples of the one or more cleaning mechanisms are disclosed in greater detail in International Publication No. WO/2016/025465 (filed August 11, 2016)(disclosing "Systems and Methods for Cleaning an Endoscopic Instrument"). The visualization system may be implemented as hardware, firmware, software or a combination thereof which interact with or are otherwise executed by one or more computer processors, which may include the processors of a control system 112.

Display system 110 may also display an image of the surgical site and medical instruments captured by the visualization system. In some examples, teleoperated medical system 100 may configure medical instrument 104 and controls of master assembly 106 such that the relative positions of the medical instruments are similar to the relative positions of the eyes and hands of physician O. In this manner physician O can manipulate medical instrument 104 and the hand control as if viewing the workspace in substantially true presence. By true presence, it is meant that the presentation of an image is a true perspective image simulating the viewpoint of a physician that is physically manipulating medical instrument 104.

In some examples, display system 110 may present images of a surgical site recorded pre-operatively or intra-operatively using image data from imaging technology such as, computed tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. The pre-operative or intra-operative image data may be presented as two-dimensional, three-dimensional, or four-dimensional (including e.g., time based or velocity based information) images and/or as images from models created from the pre-operative or intra-operative image data sets.

In some examples, often for purposes of imaged guided surgical procedures, display system 110 may display a virtual navigational image in which the actual location of medical instrument 104 is registered (i.e., dynamically referenced) with the preoperative or concurrent images/model. This may be done to present the clinician or physician O with a virtual image of the internal surgical site from a viewpoint of medical instrument 104. In some examples, the viewpoint may be from a tip of medical instrument 104. An image of the tip of medical instrument 104 and/or other graphical or alphanumeric indicators may be superimposed on the virtual image to assist physician O controlling medical instrument 104. In some examples, medical instrument 104 may not be visible in the virtual image.

In some examples, display system 110 may display a virtual navigational image in which the actual location of medical instrument 104 is registered with preoperative or concurrent images to present the clinician or physician O with a virtual image of medical instrument 104 within the surgical site from an external viewpoint. An image of a portion of medical instrument 104 or other graphical or alphanumeric indicators may be superimposed on the virtual image to assist physician O in the control of medical instrument 104. As described herein, visual representations of data points may be rendered to display system 110. For example, measured data points, moved data points, registered data points, and other data points described herein may be displayed on display system 110 in a visual representation. The data points may be visually represented in a user interface by a plurality of points or dots on display system 110 or as a rendered model, such as a mesh or wire model created based on the set of data points. In some examples, the data points may be color coded according to the data they represent. In some examples, a visual representation may be refreshed in display system 110 after each processing operation has been implemented to alter data points.

Teleoperated medical system 100 may also include control system 112. Control system 112 includes at least one memory and at least one computer processor (not shown) for effecting control between medical instrument 104, master assembly 106, sensor system 108, and display system 110. Control system 112 also includes programmed instructions (e.g., a non-transitory machine-readable medium storing the instructions) to implement some or all of the methods described in accordance with aspects disclosed herein, including instructions for providing information to display system 110. While control system 112 is shown as a single block in the simplified schematic of FIG. 1, the system may include two or more data processing circuits with one portion of the processing optionally being performed on or adjacent to teleoperational manipulator assembly 102, another portion of the processing being performed at master assembly 106, and/or the like. The processors of control system 112 may execute instructions comprising instruction corresponding to processes disclosed herein and described in more detail below. Any of a wide variety of centralized or distributed data processing architectures may be employed. Similarly, the programmed instructions may be implemented as a number of separate programs or subroutines, or they may be integrated into a number of other aspects of the teleoperational systems described herein. In one embodiment, control system 112 supports wireless communication protocols such as Bluetooth, IrDA, HomeRF, IEEE 802.11, DECT, and Wireless Telemetry.

In some examples, control system 112 may receive force and/or torque feedback from medical instrument 104. Responsive to the feedback, control system 112 may transmit signals to master assembly 106. In some examples, control system 112 may transmit signals instructing one or more actuators of teleoperational manipulator assembly 102 to move medical instrument 104. Medical instrument 104 may extend into an internal surgical site within the body of patient P via openings in the body of patient P. Any suitable conventional and/or specialized actuators may be used. In some examples, the one or more actuators may be separate from, or integrated with, teleoperational manipulator assembly 102. In some examples, the one or more actuators and teleoperational manipulator assembly 102 are provided as part of a teleoperational cart positioned adjacent to patient P and operating table T.

Control system 112 may optionally further include a virtual visualization system to provide navigation assistance to physician O when controlling medical instrument 104 during an image-guided surgical procedure. Virtual navigation using the virtual visualization system may be based upon reference to an acquired preoperative or intraoperative dataset of anatomic passageways. The virtual visualization system processes images of the surgical site imaged using imaging technology such as computerized tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. Software, which may be used in combination with manual inputs, is used to convert the recorded images into segmented two dimensional or three dimensional composite representation of a partial or an entire anatomic organ or anatomic region. An image data set is associated with the composite representation. The composite representation and the image data set describe the various locations and shapes of the passageways and their connectivity. The images used to generate the composite representation may be recorded preoperatively or intra-operatively during a clinical procedure. In some examples, a virtual visualization system may use standard representations (i.e., not patient specific) or hybrids of a standard representation and patient specific data. The composite representation and any virtual images generated by the composite representation may represent the static posture of a deformable anatomic region during one or more phases of motion (e.g., during an inspiration/ expiration cycle of a lung).

During a virtual navigation procedure, sensor system 108 may be used to compute an approximate location of medical instrument 104 with respect to the anatomy of patient P. The location can be used to produce both macro-level (external) tracking images of the anatomy of patient P and virtual internal images of the anatomy of patient P. The system may implement one or more electromagnetic (EM) sensor, fiber optic sensors, and/or other sensors to register and display a medical implement together with preoperatively recorded surgical images. , such as those from a virtual visualization system, are known. For example U.S. Patent Application No. 13/107,562 (filed May 13, 2011) (disclosing "Medical System Providing Dynamic Registration of a Model of an Anatomic Structure for Image-Guided Surgery") discloses one such system. Teleoperated medical system 100 may further include optional operations and support systems (not shown) such as illumination systems, steering control systems, irrigation systems, and/or suction systems. In some examples, teleoperated medical system 100 may include more than one teleoperational manipulator assembly and/or more than one master assembly. The exact number of teleoperational manipulator assemblies will depend on the surgical procedure and the space constraints within the operating room, among other factors. Master assembly 106 may be collocated or they may be positioned in separate locations. Multiple master assemblies allow more than one operator to control one or more teleoperational manipulator assemblies in various combinations.

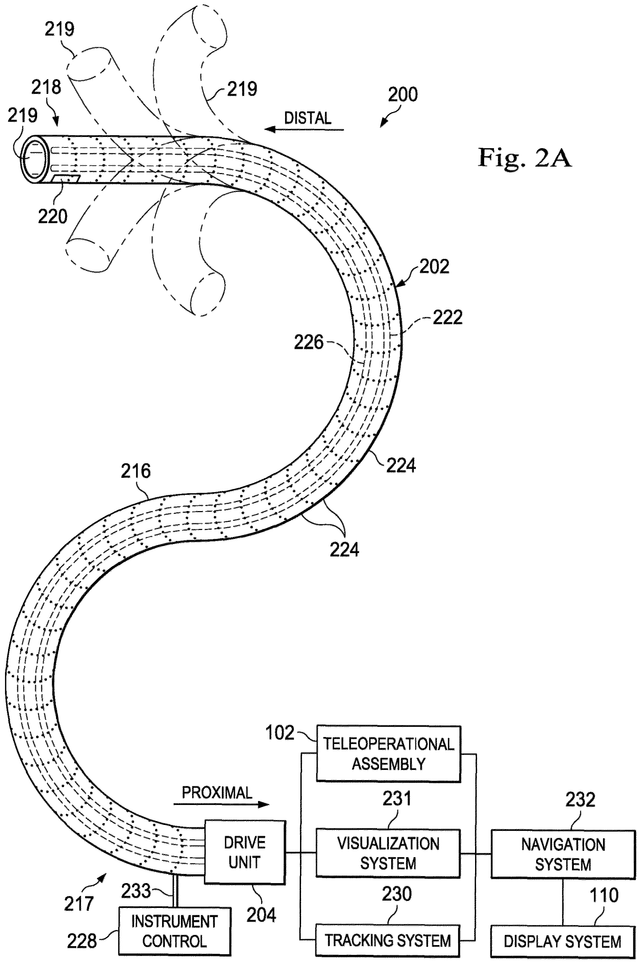

FIG. 2A is a simplified diagram of a medical instrument system 200 according to some non-claimed examples. In some examples, medical instrument system 200 may be used as medical instrument 104 in an image-guided medical procedure performed with teleoperated medical system 100. In some examples, medical instrument system 200 may be used for non-teleoperational exploratory procedures or in procedures involving traditional manually operated medical instruments, such as endoscopy. Optionally medical instrument system 200 may be used to gather (i.e., measure) a set of data points corresponding to locations within anatomic passageways of a patient, such as patient P.

Medical instrument system 200 includes elongate device 202, such as a flexible catheter, coupled to a drive unit 204. Elongate device 202 includes a flexible body 216 having proximal end 217 and distal end or tip portion 218. In some examples, flexible body 216 has an approximately 3 mm outer diameter. Other flexible body outer diameters may be larger or smaller.

Medical instrument system 200 further includes a tracking system 230 for determining the position, orientation, speed, velocity, pose, and/or shape of distal end 218 and/or of one or more segments 224 along flexible body 216 using one or more sensors and/or imaging devices as described in further detail below. The entire length of flexible body 216, between distal end 218 and proximal end 217, may be effectively divided into segments 224. If medical instrument system 200 is consistent with medical instrument 104 of a teleoperated medical system 100, tracking system 230. Tracking system 230 may optionally be implemented as hardware, firmware, software or a combination thereof which interact with or are otherwise executed by one or more computer processors, which may include the processors of control system 112 in FIG. 1.

Tracking system 230 may optionally track distal end 218 and/or one or more of the segments 224 using a shape sensor 222. Shape sensor 222 may optionally include an optical fiber aligned with flexible body 216 (e.g., provided within an interior channel (not shown) or mounted externally). In one example, the optical fiber has a diameter of approximately 200 µm. In other examples, the dimensions may be larger or smaller. The optical fiber of shape sensor 222 forms a fiber optic bend sensor for determining the shape of flexible body 216. In one alternative, optical fibers including Fiber Bragg Gratings (FBGs) are used to provide strain measurements in structures in one or more dimensions. Various systems and methods for monitoring the shape and relative position of an optical fiber in three dimensions are described in U.S. Patent Application No. 11/180,389 (filed July 13, 2005) (disclosing "Fiber optic position and shape sensing device and method relating thereto"); U.S. Patent Application No. 12/047,056 (filed on Jul. 16, 2004) (disclosing "Fiber-optic shape and relative position sensing"); and U.S. Patent No. 6,389,187 (filed on Jun. 17, 1998) (disclosing "Optical Fibre Bend Sensor"),. Sensors in some examples may employ other suitable strain sensing techniques, such as Rayleigh scattering, Raman scattering, Brillouin scattering, and Fluorescence scattering. In some embodiments, the shape of the elongate device may be determined using other techniques. For example, a history of the distal end pose of flexible body 216 can be used to reconstruct the shape of flexible body 216 over the interval of time. In some examples, tracking system 230 may optionally and/or additionally track distal end 218 using a position sensor system 220. Position sensor system 220 may be a component of an EM sensor system with position sensor system 220 including one or more conductive coils that may be subjected to an externally generated electromagnetic field. Each coil of the EM sensor system then produces an induced electrical signal having characteristics that depend on the position and orientation of the coil relative to the externally generated electromagnetic field. In some examples, position sensor system 220 may be configured and positioned to measure six degrees of freedom, e.g., three position coordinates X, Y, Z and three orientation angles indicating pitch, yaw, and roll of a base point or five degrees of freedom, e.g., three position coordinates X, Y, Z and two orientation angles indicating pitch and yaw of a base point. Further description of a position sensor system is provided in U.S. Patent No. 6,380,732 (filed August 11, 1999) (disclosing "Six-Degree of Freedom Tracking System Having a Passive Transponder on the Object Being Tracked"),.

In some examples, tracking system 230 may alternately and/or additionally rely on historical pose, position, or orientation data stored for a known point of an instrument system along a cycle of alternating motion, such as breathing. This stored data may be used to develop shape information about flexible body 216. In some examples, a series of positional sensors (not shown), such as electromagnetic (EM) sensors similar to the sensors in position sensor 220 may be positioned along flexible body 216 and then used for shape sensing. In some examples, a history of data from one or more of these sensors taken during a procedure may be used to represent the shape of elongate device 202, particularly if an anatomic passageway is generally static.

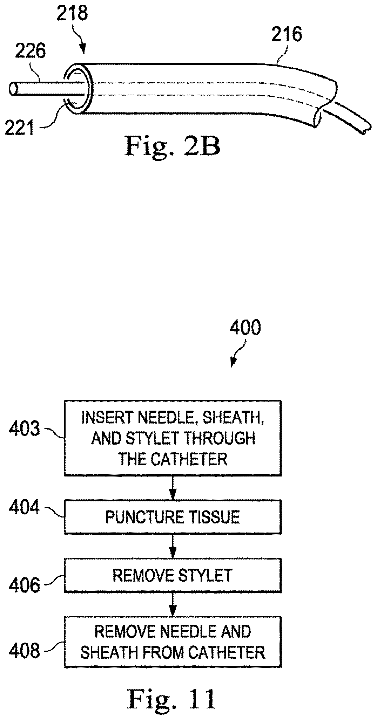

Flexible body 216 includes a channel 221 sized and shaped to receive a medical instrument 226. FIG. 2B is a simplified diagram of flexible body 216 with medical instrument 226 extended according to some examples. In some examples, medical instrument 226 may be used for procedures such as surgery, biopsy, ablation, illumination, irrigation, or suction. Medical instrument 226 can be deployed through channel 221 of flexible body 216 and used at a target location within the anatomy. Medical instrument 226 may include, for example, image capture probes, biopsy instruments, laser ablation fibers, and/or other surgical, diagnostic, or therapeutic tools. Medical tools may include end effectors having a single working member such as a scalpel, a blunt blade, an optical fiber, an electrode, and/or the like. Other end effectors may include, for example, forceps, graspers, scissors, clip appliers, and/or the like. Other end effectors may further include electrically activated end effectors such as electrosurgical electrodes, transducers, sensors, and/or the like. In various examples, medical instrument 226 is a biopsy instrument, which may be used to remove sample tissue or a sampling of cells from a target anatomic location. Medical instrument 226 may be used with an image capture probe also within flexible body 216. In various examples, medical instrument 226 may be an image capture probe that includes a distal portion with a stereoscopic or monoscopic camera at or near distal end 218 of flexible body 216 for capturing images (including video images) that are processed by a visualization system 231 for display and/or provided to tracking system 230 to support tracking of distal end 218 and/or one or more of the segments 224. The image capture probe may include a cable coupled to the camera for transmitting the captured image data. In some examples, the image capture instrument may be a fiber-optic bundle, such as a fiberscope, that couples to visualization system 231. The image capture instrument may be single or multispectral, for example capturing image data in one or more of the visible, infrared, and/or ultraviolet spectrums. Alternatively, medical instrument 226 may itself be the image capture probe. Medical instrument 226 may be advanced from the opening of channel 221 to perform the procedure and then retracted back into the channel when the procedure is complete. Medical instrument 226 may be removed from proximal end 217 of flexible body 216 or from another optional instrument port (not shown) along flexible body 216. The movement of the medical instrument 226 relative to the flexible body 216 may be controlled by a manual device such as an operator controlled handle or may be driven by teleoperational control. In one example, a tool control device 228 is coupled to a proximal end of the instrument 226 to control movement of the instrument 226 within the channel 221. A port 233 coupled to the flexible body 216 allows through passage of the instrument 226 into the channel 221. In one example the tool control device may be a handle at the proximal end of the tool.

Medical instrument 226 may additionally house cables, linkages, or other actuation controls (not shown) that extend between its proximal and distal ends to controllably the bend distal end of medical instrument 226. Steerable instruments are described in detail in U.S. Patent No. 7,316,681 (filed on Oct. 4, 2005) (disclosing "Articulated Surgical Instrument for Performing Minimally Invasive Surgery with Enhanced Dexterity and Sensitivity") and U.S. Patent Application No. 12/286,644 (filed Sept. 30, 2008) (disclosing "Passive Preload and Capstan Drive for Surgical Instruments").

Flexible body 216 may also house cables, linkages, or other steering controls (not shown) that extend between drive unit 204 and distal end 218 to controllably bend distal end 218 as shown, for example, by broken dashed line depictions 219 of distal end 218. In some examples, at least four cables are used to provide independent "up-down" steering to control a pitch of distal end 218 and "left-right" steering to control a yaw of distal end 281. Steerable elongate devices are described in detail in U.S. Patent Application No. 13/274,208 (filed Oct. 14, 2011) (disclosing "Catheter with Removable Vision Probe"). In examples in which medical instrument system 200 is actuated by a teleoperational assembly, drive unit 204 may include drive inputs that removably couple to and receive power from drive elements, such as actuators, of the teleoperational assembly. In some examples, medical instrument system 200 may include gripping features, manual actuators, or other components for manually controlling the motion of medical instrument system 200. Elongate device 202 may be steerable or, alternatively, the system may be non-steerable with no integrated mechanism for operator control of the bending of distal end 218. In some examples, one or more lumens, through which medical instruments can be deployed and used at a target surgical location, are defined in the walls of flexible body 216.

In some examples, medical instrument system 200 may include a flexible bronchial instrument, such as a bronchoscope or bronchial catheter, for use in examination, diagnosis, biopsy, or treatment of a lung. Medical instrument system 200 is also suited for navigation and treatment of other tissues, via natural or surgically created connected passageways, in any of a variety of anatomic systems, including the colon, the intestines, the kidneys and kidney calices, the brain, the heart, the circulatory system including vasculature, and/or the like.

The information from tracking system 230 may be sent to a navigation system 232 where it is combined with information from visualization system 231 and/or the preoperatively obtained models to provide the physician or other operator with real-time position information. In some examples, the real-time position information may be displayed on display system 110 of FIG. 1 for use in the control of medical instrument system 200. In some examples, control system 116 of FIG. 1 may utilize the position information as feedback for positioning medical instrument system 200. Various systems for using fiber optic sensors to register and display a surgical instrument with surgical images are provided in U.S. Patent Application No. 13/107,562, filed May 13, 2011, disclosing, "Medical System Providing Dynamic Registration of a Model of an Anatomic Structure for Image-Guided Surgery," .

In some examples, medical instrument system 200 may be teleoperated within medical system 100 of FIG. 1. In some examples, teleoperational manipulator assembly 102 of FIG.

1 may be replaced by direct operator control. In some examples, the direct operator control may include various handles and operator interfaces for hand-held operation of the instrument.

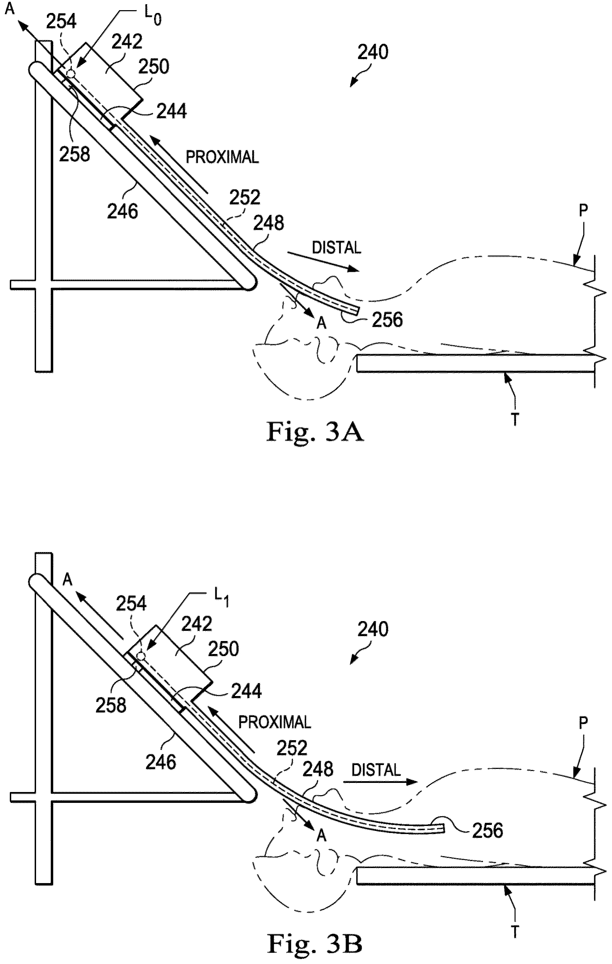

FIGS. 3A and 3B are simplified diagrams of side views of a patient coordinate space including a medical instrument mounted on an insertion assembly according to some non-claimed examples. As shown in FIGS. 3A and 3B, a surgical environment 240 includes a patient P is positioned on the table T of FIG. 1. Patient P may be stationary within the surgical environment in the sense that gross patient movement is limited by sedation, restraint, and/or other means. Cyclic anatomic motion including respiration and cardiac motion of patient P may continue, unless patient is asked to hold his or her breath to temporarily suspend respiratory motion. Accordingly, in some examples, data may be gathered at a specific, phase in respiration, and tagged and identified with that phase. In some examples, the phase during which data is collected may be inferred from physiological information collected from patient P. Within surgical environment 240, a point gathering instrument 242 is coupled to an instrument carriage 244. In some examples, point gathering instrument 242 may use EM sensors, shape-sensors, and/or other sensor modalities. Instrument carriage 244 is mounted to an insertion stage 246 fixed within surgical environment 240. Alternatively, insertion stage 246 may be movable but have a known location (e.g., via a tracking sensor or other tracking device) within surgical environment 240. Instrument carriage 244 may be a component of a teleoperational manipulator assembly (e.g., teleoperational manipulator assembly 102) that couples to point gathering instrument 242 to control insertion motion (i.e., motion along the A axis) and, optionally, motion of a distal end 256 of an elongate device 248 in multiple directions including yaw, pitch, and roll. Instrument carriage 244 or insertion stage 246 may include actuators, such as servomotors, (not shown) that control motion of instrument carriage 244 along insertion stage 246.

Elongate device 248 is coupled to an instrument body 250. Instrument body 250 is coupled and fixed relative to instrument carriage 244. In some examples, an optical fiber shape sensor 252 is fixed at a proximal point 254 on instrument body 250. In some examples, proximal point 254 of optical fiber shape sensor 252 may be movable along with instrument body 250 but the location of proximal point 254 may be known (e.g., via a tracking sensor or other tracking device). Shape sensor 252 measures a shape from proximal point 254 to another point such as distal end 256 of elongate device 248. Point gathering instrument 242 may be substantially similar to medical instrument system 200.

A position measuring device 258 provides information about the position of instrument body 250 as it moves on insertion stage 246 along an insertion axis A. Position measuring device 258 may include resolvers, encoders, potentiometers, and/or other sensors that determine the rotation and/or orientation of the actuators controlling the motion of instrument carriage 244 and consequently the motion of instrument body 250. In some examples, insertion stage 246 is linear. In some examples, insertion stage 246 may be curved or have a combination of curved and linear sections.

FIG. 3A shows instrument body 250 and instrument carriage 244 in a retracted position along insertion stage 246. In this retracted position, proximal point 254 is at a position L₀ on axis A. In this position along insertion stage 246 an A component of the location of proximal point 254 may be set to a zero and/or another reference value to provide a base reference to describe the position of instrument carriage 244, and thus proximal point 254, on insertion stage 246. With this retracted position of instrument body 250 and instrument carriage 244, distal end 256 of elongate device 248 may be positioned just inside an entry orifice of patient P. Also in this position, position measuring device 258 may be set to a zero and/or the another reference value (e.g., 1=0). In FIG. 3B, instrument body 250 and instrument carriage 244 have advanced along the linear track of insertion stage 246 and distal end 256 of elongate device 248 has advanced into patient P. In this advanced position, the proximal point 254 is at a position L₁ on the axis A. In some examples, encoder and/or other position data from one or more actuators controlling movement of instrument carriage 244 along insertion stage 246 and/or one or more position sensors associated with instrument carriage 244 and/or insertion stage 246 is used to determine the position Lₓ of proximal point 254 relative to position L₀. In some examples, position Lₓ may further be used as an indicator of the distance or insertion depth to which distal end 256 of elongate device 248 is inserted into the passageways of the anatomy of patient P.

Medical tools used with the flexible body of the catheter system should be flexible enough to navigate the tight turns and bends in the patient anatomical passageways traced by the catheter system. For example, the catheter system may follow a curve radius of 13mm or less.

However, some medical tools require localized rigidity to perform their intended medical function. A medical tool such as a biopsy instrument, for example, may require a rigid distal tip portion to puncture tissue and to allow penetration of dense or hardened tissue. Described below are medical tools, including biopsy instruments that are pliant enough to permit passage through tortuous passageways, while still providing sufficient distal rigidity to extend from the catheter along a generally straight trajectory aligned with the orientation of the distal end of the catheter. The medical tools described herein may be used with the medical instrument system 200, including with catheter system 202, or with another guidance system such as a bronchoscope.

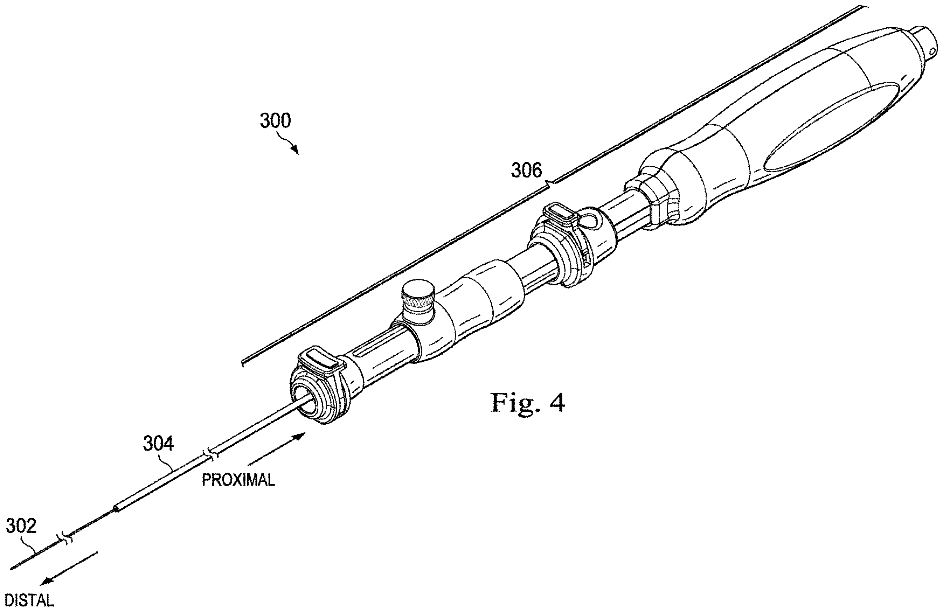

FIG. 4 illustrates a medical tool 300 (e.g. a medical tool 226). In this embodiment medical tool 300 is a biopsy tool, but in alternative embodiments, various other tools including treatment (e.g., ablation) or imaging tools may be used with the principles described herein. The biopsy tool 300 includes a biopsy needle 302 extended from a sheath 304. The biopsy needle 302 and sheath 304 are coupled to a handle assembly 306 that allows a user to move the needle within and relative to the sheath. In one example, the biopsy needle is a 19 gauge needle having an approximately 1mm outer diameter that extends within a sheath having an approximately 1.75mm outer diameter. In other examples, the smaller or larger sized needles may be used with the principles of this disclosure. In some examples, the biopsy needle may be extended approximately 3cm from the distal end of the sheath 304 to extract biopsy tissue.

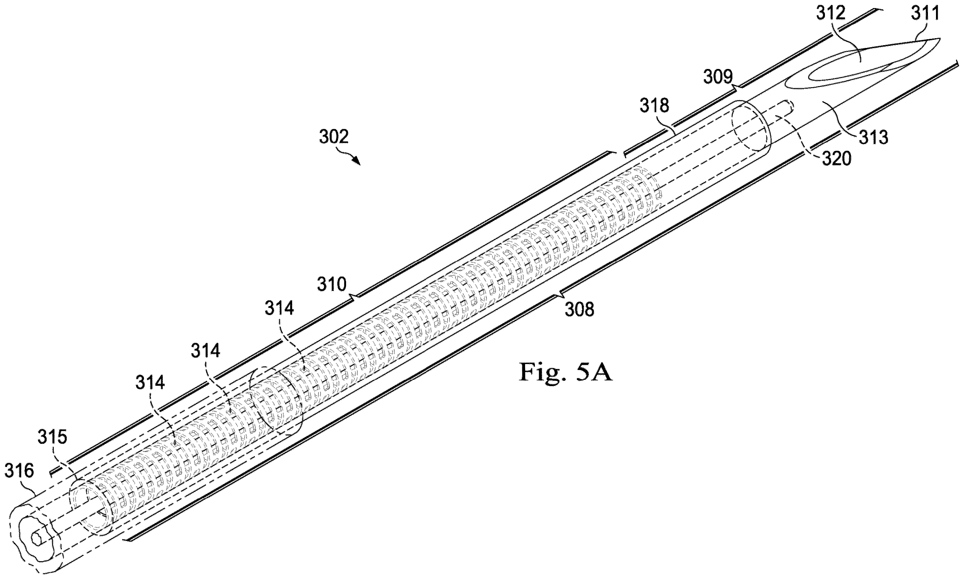

FIG. 5A illustrates a distal section 308 of the needle 302. The distal section 308 may be formed from a relatively rigid material including a metal or a rigid polymer. In this embodiment, the distal section 308 is formed from a stainless steel hypotube. The distal section 308 includes rigid portion 309 and flexible portion 310. The rigid portion 309 includes a cutting surface 311 surrounding an opening 312 to a channel 313 that extends through the distal section 308.

The flexible portion 310 includes one or more slits 314 that extend through the wall of the distal section 308 to the channel 313, allowing the flexible portion to bend. The slits 314 may have a variety of circumferential configurations (as described below) that extend along the longitudinal length of the flexible portion 310. For example, a single spiral slit may extend around the length of the flexible portion. Alternatively, an interrupted spiral slit pattern or an interrupted slit pattern, having a plurality of pitched or perpendicular slits (relative to the central longitudinal axis through the channel 313), may be formed in the flexible portion 310. The slits 314 may be formed by laser cutting of the tubular body of the distal section 308.

In one example, the rigid portion 309 has a lancet point with a twelve degree angle. In one example, the rigid portion may be approximately 5mm, but longer or shorter rigid portions may be suitable. In one example, the flexible portion 310 may be approximately 3 cm (1.2 inches ) to 4.5 cm ( 1.8 inches ) long, but longer or shorter flexible portions may be suitable. In alternative examples, the needle may have a side opening through a lateral wall of the rigid portion to collect sheared tissue biopsy samples.

A flexible jacket 318 (also sleeve 318) extends around and is coupled with the flexible portion 310. The jacket may be formed from, for example, a polymer material and interlocks with the flexible portion 310 by extending into the slits. The flexible jacket 318 can be impervious to fluid and can act as a flexible barrier to fluid flow through the slits 314. For example, if a vacuum is applied along the channel 313 to pull tissue and bodily fluids in through the opening 312, the jacket 318 prevents flow of the tissue and fluids out of the channel 313 and through the wall of the flexible portion 310. In one example, the jacket 318 may be formed from a thin polyethylene terephthalate (PET) heat shrink material that may be molded on to the flexible portion 310. The heat shrink material interlocks with the flexible portion by flowing into the slits 314 and when cooled, frictionally anchoring the jacket to the flexible portion. In other words, when the jacket is heated, it shrinks into the slits and when cooled, is interlocked with the slits. In other examples, polyamide, polyimide, Pebax, polytetrafluorotheylene (PTFE), fluorinated ethylene propylene (FEP) and polyurethane may be used as the jacket material. In another embodiment, a thermoplastic tubing such as PEBAX with a low durometer (e.g. 35 D) may be molded (e.g., via thermal flow) into the slits, closing off the slits to allow a vacuum, but flexible enough to allow bending. During the thermal flow, a mandrel may be used in the ID of the needle to prevent the material flow into the channel.

The needle 302 also includes a flexible shaft 316 coupled to a proximal end of the distal section 308. The shaft 316 extends into a portion of the slits. In one example, the shaft 316 may be formed from a flexible polymer that is coupled to the distal section 308 by melting into the slits 314 to mechanically lock the shaft to the distal section 308 of the needle 302. At the joint 315 where the shaft 316 and distal section 308 are coupled, the jacket 318 is sandwiched between the shaft and the flexible portion 310. Proximal of the rigid portion 309 the needle 302 is pliant due to the flexible shaft 316 and flexible portion 310, allowing passage of the needle through tight bends in narrow anatomical passageways.

When the needle 302 passes through a tight bend in the catheter 202, the jacket 318 may develop a set curve or permanently bent shape that that does not does not straighten completely when the needle 302 advances distally from the catheter 202. This set curve may bias the emerging needle to curve away from the orientation of the distal end of the guiding catheter. Biopsy accuracy may rely upon a predictable straight-line needle trajectory aligned with the orientation of the distal end of the catheter. To straighten the needle 302 that has developed a set curve, a stylet 320 may extend through the channel 313 of the needle 302 into the rigid portion 309. When the rigid portion 309 and flexible portion 310 advance from the catheter during a biopsy procedure, the stylet 320 directs the rigid portion in a straight trajectory aligned with the orientation of the distal end of the guiding catheter. The stylet may be made of a super elastic material that provides a reversible physical response to an applied stress, which can be enabled by a material phase transformation. Examples of superelastic materials include various shape-memory alloys including Nitinol. A stylet made of a superelastic material does not permanently retain a bent shape but rather returns to a pre-established straightened configuration after traversing a curve. Other wire materials such as hard tempered stainless steel may be used to form the stylet but a small diameter stainless steel stylet may be needed to prevent a permanent bend in the stylet. Such a small diameter wire would have a lower straightening force than the Nitinol wire and thus may not be as effective in straightening the distal end of the needle. The stylet 320 may extend through the needle 302 while puncturing tissue and may be removed to allow collection of tissue within the channel of the needle.

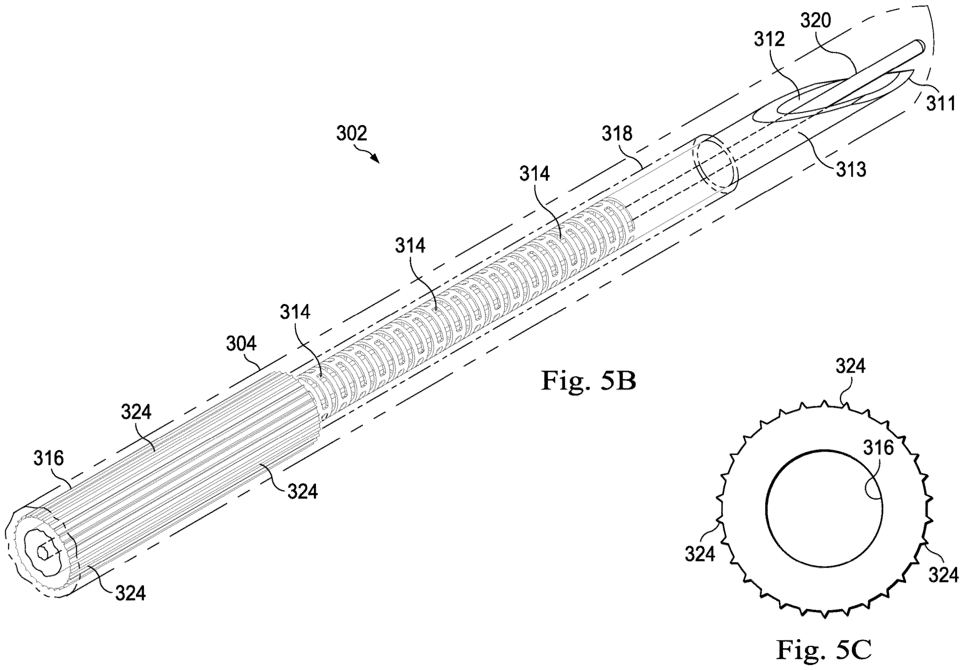

As illustrated in FIG. 5B, the sheath 304 protects the point of the needle 302 from damage while being inserted through the catheter and protects the internal surface of the guiding catheter channel from becoming damaged by the sharp tip of the needle. The sheath 304 is positioned around the point of the needle 302 while the pair are advanced together through the catheter to a target anatomical location. Once the sheath 304 and needle 302 are fully advanced and positioned at the distal end of the catheter, the rigid portion 309 is extended distally from the sheath 304 and the catheter toward the target tissue. After the biopsy, the needle 302 is retracted into the sheath 304, and the needle and sheath are withdrawn from the catheter.

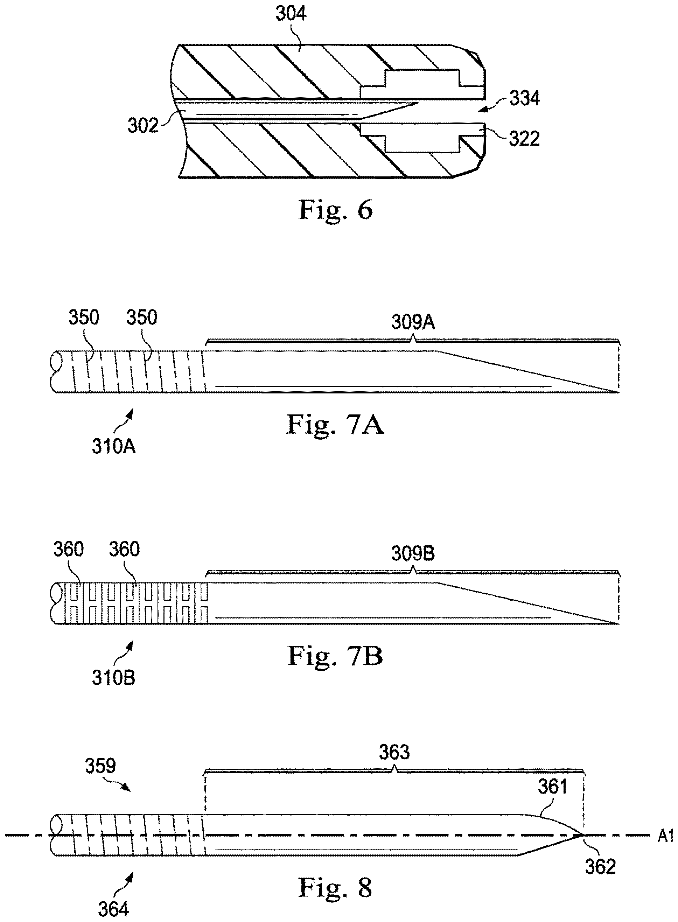

The sheath 304 may be formed of a flexible tubular shaped polymer. As shown in FIG. 6, the central channel 334 of the sheath 304 may be fitted near a distal portion with a guard member 322 that prevents the point of the needle 302 from gouging the inner surface of the sheath or otherwise becoming caught in the sheath. It can also provide an otherwise flexible sheath with a stiffer portion which aids in guiding the needle in a straight trajectory when advancing past the sheath distal tip. The guard member 322 may be formed or stainless steel or another type of radiopaque material that may be visualized on fluoroscopic images. In alternative embodiments, the guard member 322 may be eliminated and the distal end of the sheath 304 may be formed of a hardened plastic providing stiffness, such as fiberglass reinforced plastic embedded with barium sulfate to additionally provide radiopacity.

When traversing tight bends, large amounts of friction may develop between the outer surface of the needle 302 and the inner surface of the sheath 304 that prevent or limit movement of the needle relative to the sheath. Referring again to FIG. 5B, surface features 324 (also "surface discontinuities 324") may be formed on the outer surface of the needle 302 to minimize the surface area making contact between the outer surface of the needle and the inner surface of the sheath, thus reducing the friction. In the examples of FIGS. 5B and 5C, the surface features 324 are longitudinal ribs formed on the outer surface of the shaft 316. In these examples, the longitudinal ribs that form the features 324 extend generally parallel to a longitudinal axis A through the needle 302. Other types of surface discontinuities may be used to reduce friction including ridges and spiked projections. The surface discontinuities may be integrally formed on the shaft of the needle or on a sleeve that fits over the needle. The sleeve may be formed of a polymer and may be coupled to the needle by heating the sleeve so that the sleeve polymer flows into the slits 314 to form a mechanical coupling of the cooled polymer within the slits. Thus, the sleeve also acts as a barrier to prevent the flow of fluids through the slits. In various examples, the j acket 318 may be removed from the length of the slits 314 that are bonded and sealed by the sleeve. The sleeve may be formed from a variety of materials that reduce friction and provide radiopaque properties. In one example, the sleeve may be formed from a polymer mix such as polymide 12 and barium sulfate. In alternative examples, the surface of the needle 302 may be smooth and the inner surface of the sheath may be formed with surface discontinuities to prevent friction between the sheath and the needle.

FIG. 7A is a side view of a distal end of a biopsy tool with a rigid portion 309A, which is substantially similar to rigid portion 309 and a flexible portion 310A including a slit pattern 350. In this example, the rigid portion 309A has a lancet point beveled from one side of a hypotube. In this example, the slit pattern 350 is an interrupted spiral pattern. A continuous spiral pattern may allow deformable stretch or bend when the needle navigates tight bends, but an interrupted spiral pattern creates bending flexibility while limiting linear deformation and stretch. The angle of the spiral pattern may vary to provide a desired flexibility. For example, the slit pattern may have approximately 2.5 cuts per rotation with 120° cut and 24° uncut with a slight pitch of approximately 0.006 inches.

FIG. 7B is a side view of a distal end of a biopsy tool with a rigid portion 309B substantially similar to rigid portion 309 and a flexible portion 310B including a slit pattern 360. In this example, the slit pattern 360 is a perpendicular slit pattern in which adjacent slits are alternated with a rotation of 90°. Each slit is approximately perpendicular to the longitudinal axis of the needle. The spacing of the slit pattern may vary to provide a desired flexibility.

FIG. 8 is a side view of a distal end of a biopsy tool 359 with a curved distal end 361 and a distal point 362 centered along a centerline A1 through the biopsy tool. The curved distal end 361 and distal point 362 are part of a rigid portion 363 of the biopsy tool 359. As compared to a lancet point that has a tendency to cause the tool to curve away from the beveled edge when advancing, placing the point 362 along the needle centerline A1 causes the needle to advance in a straight trajectory through tissue. The rigid portion 363 of the tool is coupled to a flexible portion 364 which may be the same or similar to any of the flexible, slitted portions described above. As compared to a lancet style needle that may curve and burrow into the sheath wall, displace a piece of the sheath into the patient, or become damaged and ineffective in penetrating tissue, the biopsy tool 359 with a centered point reduces the likelihood of catching on the inner wall of the sheath, especially when navigating tight curves.

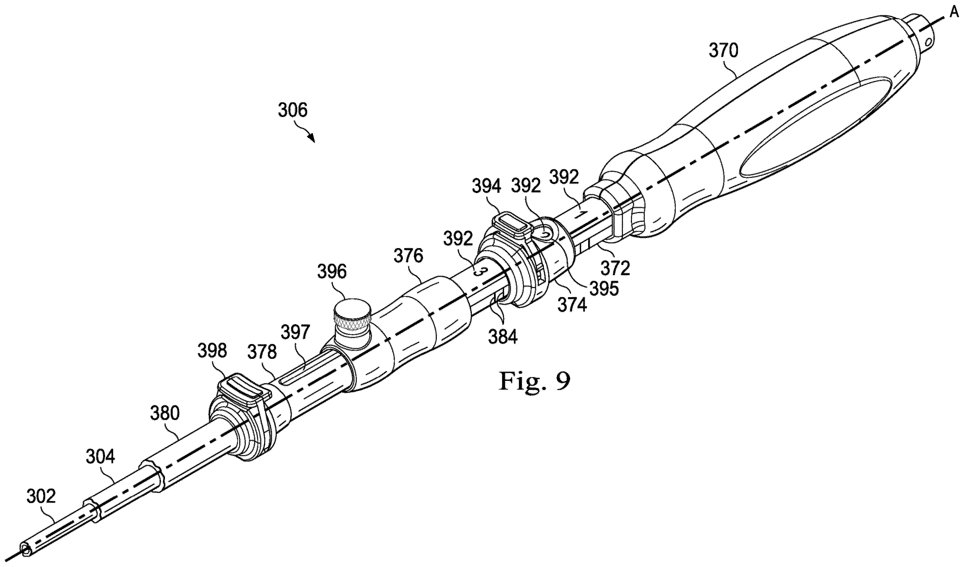

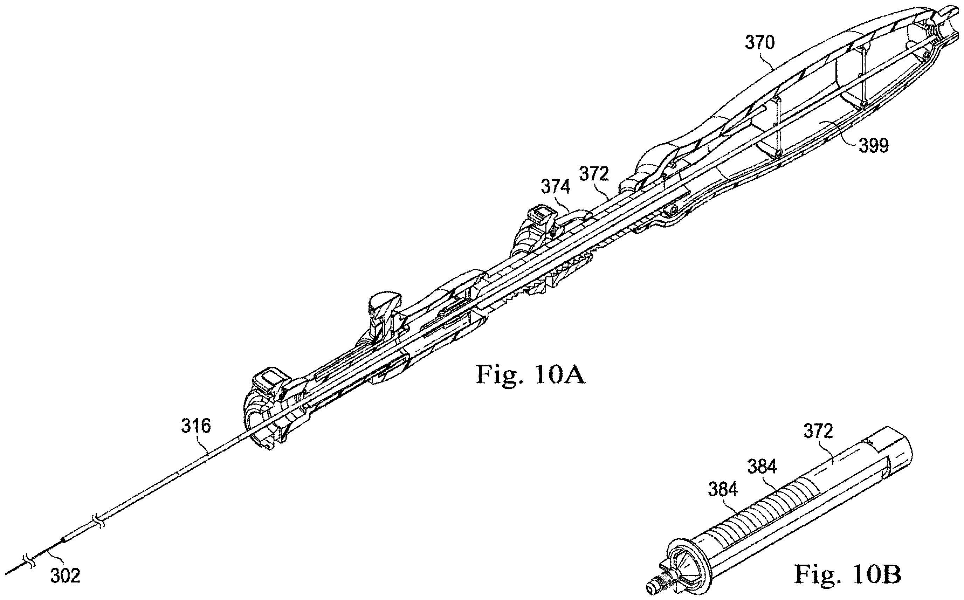

FIG. 9 illustrates the handle assembly 306 which is an example of a tool control device 228. Handle assembly 306 includes a handle body 370 slidingly coupled to a hollow shaft 372. The hollow shaft 372 extends through a needle stop 374 and is fixedly coupled to a hub 376. Hub 376 is slidingly coupled to a connector assembly 378. As shown in FIG. 10A, the proximal end of the needle 302 is coupled to a tube 399 which may be a metal hypotube. The tube 399 extends through the hollow shaft 372 and is fixed within the handle body 370. In one example, the tube is coupled to the handle body 370 by plastic overmolding. The proximal end of the sheath 304 is coupled to a distal end of the hollow shaft 372. A catheter port 380 (e.g. port 233) is coupled to a distal end of connector assembly 378. The catheter port 380 may be in communication with the working channel 221 of the catheter system 202.

Sliding movement of the hollow shaft 372 into and out of the handle body 370 may be restricted by the needle stop 374. Multiple stop position markings along the shaft 372 are marked with alphanumeric or graphical markings 392. As shown in FIG. 10B, spaced apart ratchet teeth 384 are arranged along an outer bottom wall of the shaft 372. A needle stop key 394 locks the needle stop 374 to the shaft 372 via the ratchet teeth 384. By depressing the needle stop key 394, the key releases from the ratchet teeth 384, allowing the needle stop 374 to be slid longitudinally along the shaft 372 to another ratchet position. After repositioning, the needle stop key 394 may be released and the needle stop 374 is locked in a different longitudinal location along the shaft 372. The handle body 370 can then be slid along the shaft 372 until the distal end of the handle body abuts the proximal end of the needle stop. As the handle body 370 moves relative to the shaft 372, the needle 302, which is fixed to the body 370, moves relative to the sheath 304, which is fixed to the shaft 372. The markings 392, visible through an opening 395 in the needle stop 374, indicate the depth of insertion of the needle 302 when the handle body 370 abuts the needle stop 374.

The longitudinal position of the connector assembly 378 relative to the hub 376 is controlled by a connector 396, such as a thumb screw, that engages a track 397 in the connector assembly. The distal end of the connector assembly 378 includes a quick connect key 398 that engages and releases the catheter port 380. Thus, the position of the catheter port 380 relative to the hub 376 may be adjusted by sliding the connector assembly 378 and repositioning it relative to the hub 376. The position of the connector assembly 378 relative to the hub may be locked by engaging the thumb screw against the track 397.

FIG. 11 illustrates a non-claimed method 400 of using the medical tool 300. The method 400 is illustrated in FIG. 11 as a set of operations or processes 403-410. Not all of the illustrated processes 403-410 may be performed in all examples of method 400. Additionally, one or more processes that are not expressly illustrated in FIG. 11 may be included before, after, in between, or as part of the processes 403-410. In some examples, one or more of the processes 403-410 are optional and may be omitted.

At a process 403, the needle 302, the sheath 304, and the stylet 320 may be advanced through a guidance system (e.g. the catheter system 202 or a bronchoscope) toward a target tissue area. The pointed distal tip of the needle 302 is covered by the sheath 304 during the advancement. At a process 404, after the needle 302, sheath 304, and stylet 320 have reached the distal end of the guidance system. As the needle and sheath assembly are advanced to hit the wall of the anatomic passageway, further insertion force causes the pointed needle to continue advancing and to puncture the wall. The blunt sheath does not puncture the wall so the needle advances past the distal end of the sheath. Alternatively, the sheath 304 may be withdrawn from the pointed distal tip before the needle and stylet are advanced. The stylet 320 helps maintain alignment of the needle trajectory with the orientation of the distal end of the guidance system. At a process 406, the stylet 320 is removed from the needle 302. Optionally, a vacuum is applied to the needle to urge tissue and fluid into the needle 302. Although the needle includes a slits in the needle wall to allow flexible bending of the needle, the slits are sealed by the j acket 318 which maintains the vacuum within the needle. At a process 408, the needle 302 and sheath 304 are removed from the catheter and the biopsied contents of the needle are removed. Optionally, with the catheter in the same orientation or in a different orientation, the processes 403-410 may be repeated to obtain multiple biopsy samples from the target tissue area.

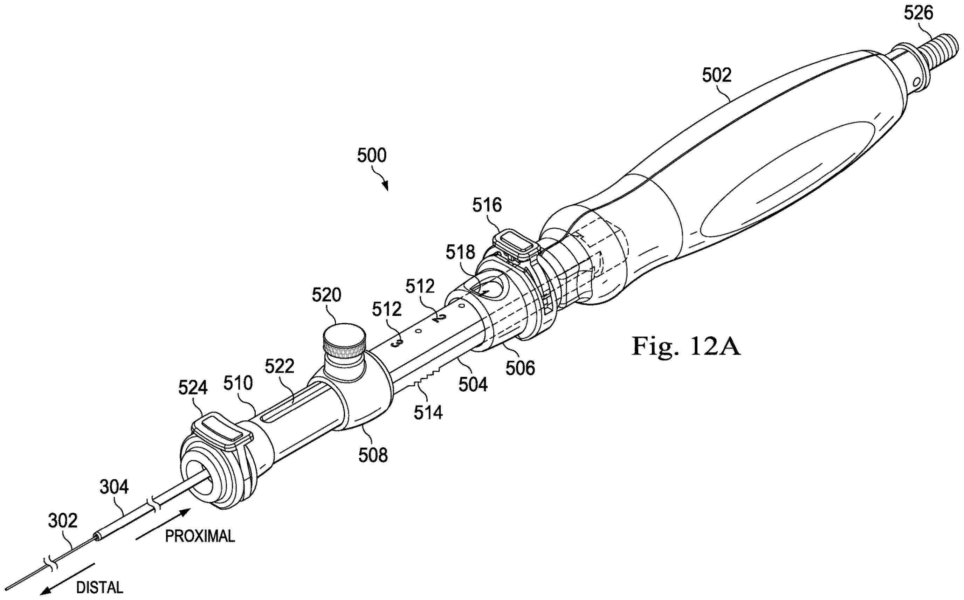

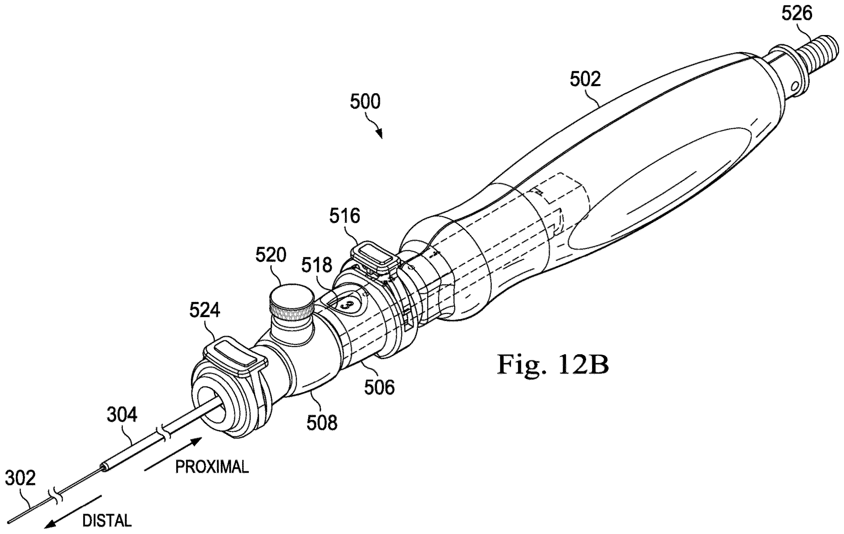

FIG. 12A illustrates a handle assembly 500 which is an example of a tool control device 228. FIG. 12A illustrates an expanded configuration of the assembly 500, and FIG. 12B illustrates a retracted configuration. Handle assembly 500 includes a handle body 502 slidingly coupled to a hollow shaft 504. The hollow shaft 504 extends through a needle stop 506 and is fixedly coupled to a hub 508. Hub 508 is slidingly coupled to a connector assembly 510. The proximal end of the biopsy needle 302 is coupled to a tube (not shown) which may be a metal hypotube. The tube extends through the hollow shaft 504 and is fixed to the handle body 502. In one example, the tube is coupled to the handle body 502 by plastic overmolding. The proximal end of the sheath 304 is coupled to a distal end of the hollow shaft 504. A catheter may be coupled to a distal end of connector assembly 510.

Sliding movement of the hollow shaft 504 into and out of the handle body 502 may be restricted by the needle stop 506. Multiple stop position markings 512 along the shaft 504 are marked with alphanumeric or graphical markings. Spaced apart ratchet teeth 514 are arranged along an outer bottom wall of the shaft 504. A needle stop key 516 locks the needle stop 506 to the shaft 504 by interfacing with the ratchet teeth 514. By depressing the needle stop key 516, the key releases from the ratchet teeth 514, allowing the needle stop 506 to be slid longitudinally along the shaft 504 to another ratchet position. After repositioning, the needle stop key 516 may be released and the needle stop 506 is locked in a different longitudinal location along the shaft 504. The handle body 502 can then be slid along the shaft 504 (with the shaft 504 sliding into the body 502) until the distal end of the handle body abuts the proximal end of the needle stop. As the handle body 502 moves relative to the shaft 504, the needle 302, which is fixed to the body 502, moves relative to the sheath 304, which is fixed to the shaft 504. The markings 512, visible through an opening 518 in the needle stop 506, indicate the depth of insertion of the needle 302 when the handle body 502 abuts the needle stop 506. In this embodiment, the opening 518 is in a distal portion of the needle stop 506, between the needle stop key 516 and the hub 508.

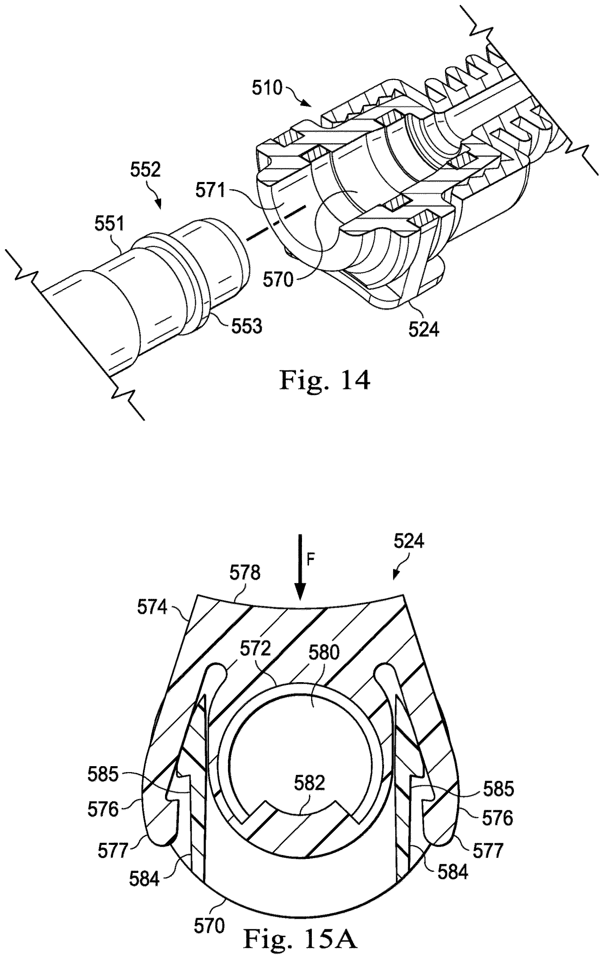

The longitudinal position of the connector assembly 510 relative to the hub 508 is controlled by a connector 520, such as a thumb screw, that engages a track 522 in the connector assembly. The distal end of the connector assembly 510 includes a connector key 524 that engages and releases the catheter. Further description of the keys 516, 524 is provided below at Fig. 14, 15A-C. Thus, the position of the catheter relative to the hub 508 may be adjusted by sliding the connector assembly 510 and repositioning it relative to the hub 508. The position of the connector assembly 510 relative to the hub may be locked by engaging the connector 520 against the track 522.

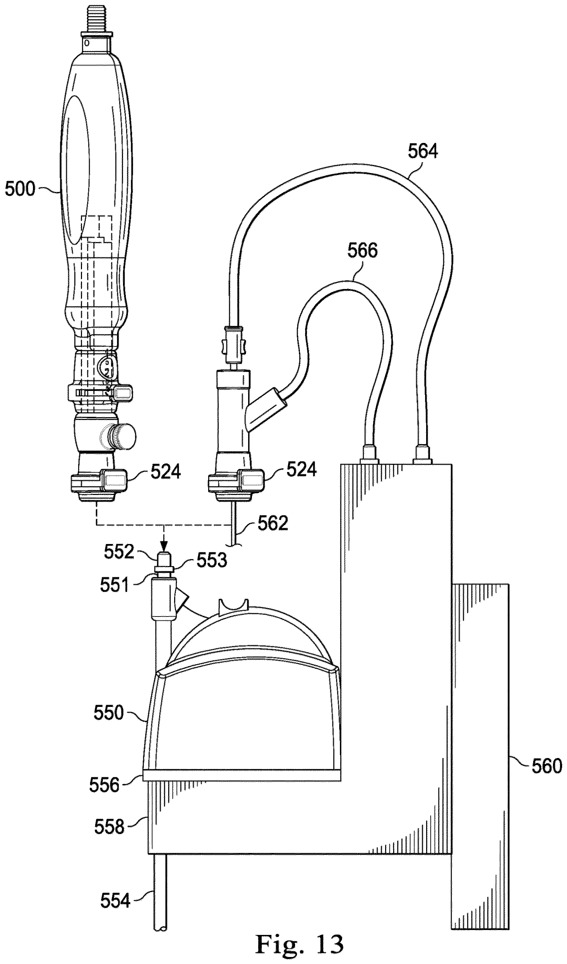

In one exemplary embodiment, the handle assembly 500 may be used to conduct a biopsy procedure as follows. The catheter (see, e.g. FIG. 13) is coupled to the connector assembly 510. With the connector 520 unlocked from the track 522, the handle body 502, shaft 504, and hub 508 may be advanced distally, as a unit, relative to the connector body and catheter to position a distal end of the sheath within a patient anatomy. The sheath 304, the biopsy needle 302, and a stylet (e.g. stylet 320) are advanced as an assembly with the handle body 502. After the sheath 304 is positioned, the connector 520 may be tightened by frictionally engaging the track 522. The stylet may be removed by pulling the stylet handle 526 from the handle body 502. A biopsy may be performed by moving the needle stop 506 along the shaft 504 until the desired insertion distance is indicated in the opening 518. To advance the needle 302 from the sheath 304, the handle body 502 and needle 302 may be pushed distally into abutment with the needle stop 506. Vacuum may be applied to capture tissue, and the needle 302 may be removed.

As shown in FIG. 13, the handle assembly 500 is one type of medical tool that can be connected to a catheter housing 550 at a catheter port 552 (e.g., port 233). The catheter port 552 includes a groove 551 and a flange 553 for coupling a connector key 524 to the port 552. The catheter housing 550 is coupled to a proximal end of a catheter 554. The catheter 554 extends through an instrument adapter 556 and an instrument carriage 558 (e.g., carriage 244). The instrument carriage 558 moves along an insertion stage 560 (e.g., 246) which may be part of a teleoperational manipulator. Other types of medical tools, including for example an image capture probe 562 or an ablation instrument may be connected to catheter port 552 to access the catheter 554 (e.g. be received within a lumen of the catheter 554). The image capture probe 562 may be communicatively coupled to the carriage 558 by a cable 564 that conveys power, image data, instruction signals or the like. The image capture probe 562 may also be coupled through the carriage 558 to a fluid source that may convey a cleaning fluid via a conduit 566 to the probe 562.

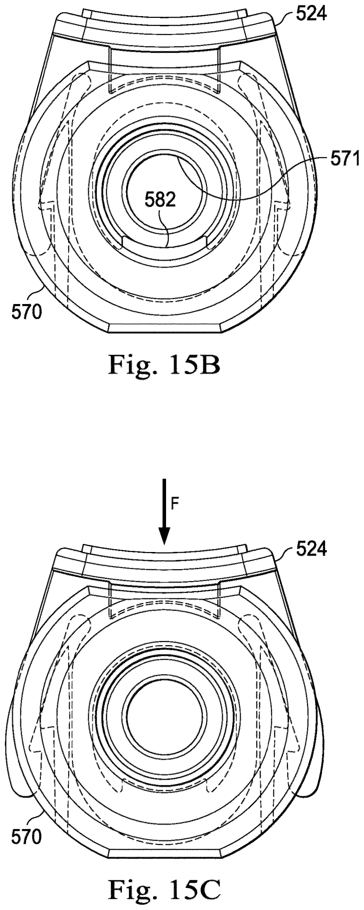

FIG. 14 illustrates a cross-sectional view of the connector assembly 510 including the connector key 524 and a connector housing 570. FIG. 15A illustrates the connector key 524 in greater detail. The connector key 524 includes a round central member 572 coupled to a link portion 574. Curved arms 576 are coupled at a top end to the link portion 574 so that the arms 576 curve around the central member 572. The bottom end of each arm 576 includes an expanded portion 577. The link portion 574 includes a finger grip surface 578 that is curved to receive a downward force F from a user's finger. The round central member 572 defines a channel 580. A projection 582 extends from the central member 572 and into the channel 580. The connector housing 570 includes a pair of guides 584 that extend on opposite sides of the central member 572, between the central member 572 and the arms 576. The connector also includes a central passage 571. The guides include a stop member 585 that limits movement of the expanded portion 577 of the arm 576 in a direction opposite the force F. As shown in FIG. 15B, without a force applied to the connector key 524, the projection 582 extends into the central passage 571. When the port 552 extends in to the central passage 571, the projection 582 projects into the groove 551 to lock the connector assembly 510 to the port 552. As shown in FIG. 15C, when the force F is applied to the connector key 524, the arms 576 bend elastically outward, away from the central member 572 and the projection 582 recessed out of the central passage 571. The movement of the arms 576 may be directed by the guides 584 and the stop members 585. With the projection recessed from the central passage 571 is also becomes removed from the groove 551, thus allowing the port 552 to become decoupled from the connector assembly 510. When the force F is removed, the arms 576 are biased to move inward toward the central member 572. To prevent the key 524 from becoming removed from the housing 570, the stop members 585 limit movement of the expanded portions 577 of the arms 576 in the direction opposite the force F. In one example, the connector assembly 510 can be included on an adaptor (not shown) configured to connect the medical tool (e.g. biopsy tool 300, image capture probe, or ablation instrument) to catheter port 552 to access the catheter 554.

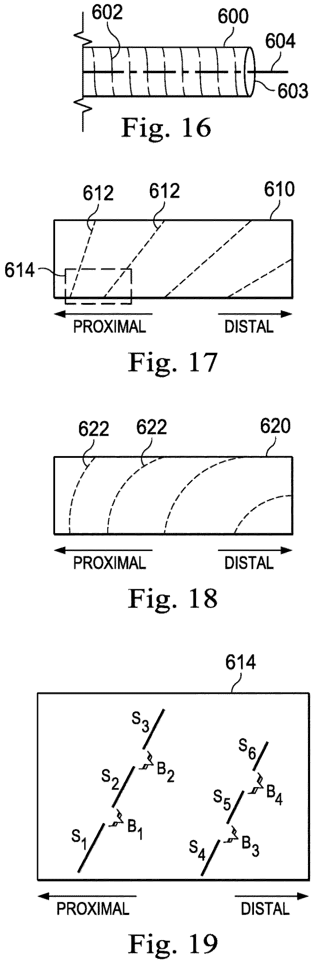

FIG. 16 illustrates a cylindrical section 600 of a biopsy needle having an irregular spiral or helical interrupted slit pattern 602 in a needle wall 603. To better depict the slit pattern, the cylindrical section 600 may be sectioned longitudinally along an axis 604 extending along the wall 603. FIG. 17 illustrates a cylindrical wall section 610 of a biopsy needle sectioned or "cut" longitudinally as shown in FIG. 16 to illustrate the slit pattern 612. As shown in FIG. 17, the wall section 610 is "unrolled" into a planar form view to illustrate a slit pattern 612. The slit pattern 612 has at least one physical parameter that is progressively altered as the pattern progresses from a proximal to a distal end of the wall section. This changing physical parameter causes the flexibility of the cylindrical section to change from the proximal end to the distal end of the wall section. For example, as shown in the enlarged view of wall section 614 in FIG. 19, the physical parameter may be a slit length. To decrease the flexibility from the proximal end to the distal end of wall section 614, slit S1 has a length greater than slit S2 which has a length greater than slit S3 which has a length greater than slit S4. The longer slits at the proximal end provide greater flexibility, and the shorter slits at the distal end provide relatively greater rigidity. As another example, as shown in the enlarged view of wall section 614 in FIG. 19, the physical parameter may be a bridge length of the bridging wall material between ends of sequential slits. To decrease the flexibility from the proximal end to the distal end of wall section 614, bridge B1 has a length shorter than bridge B2 which has a length shorter than bridge B3 which has a length shorter than bridge B4. The shorter bridge lengths at the proximal end provide greater flexibility, and the longer bridge lengths at the distal end provide relatively greater rigidity. As another example, as shown in the enlarged view of wall section 614 in FIG. 19, the physical parameter may be an angle of the slit relative to the longitudinal axis. To decrease the flexibility from the proximal end to the distal end of wall section 614, slit S1 is cut at an angle (e.g., 80°) greater than slit S2 (e.g., 79°) which is greater than the angle of slit S3 (e.g., 78°). The larger slit angles at the proximal end provide greater flexibility, and the smaller slit angles at the distal end provide relatively greater rigidity. More than one physical parameter may be altered in a needle section to provide the necessary gradation in flexibility. Other physical parameters that may be progressively altered to change the section flexibility include the pitch of the slit pattern, the width of the slits, and the angle of the helical pattern. To provide a smooth helical curve, the slits in the slit pattern may be cut on a curve which may be progressively altered. FIG. 18 illustrates a wall section 620 having a curved slit pattern 622 with each cycle of the helical rotation progressively increased from the proximal end of the section to the distal end of the section.

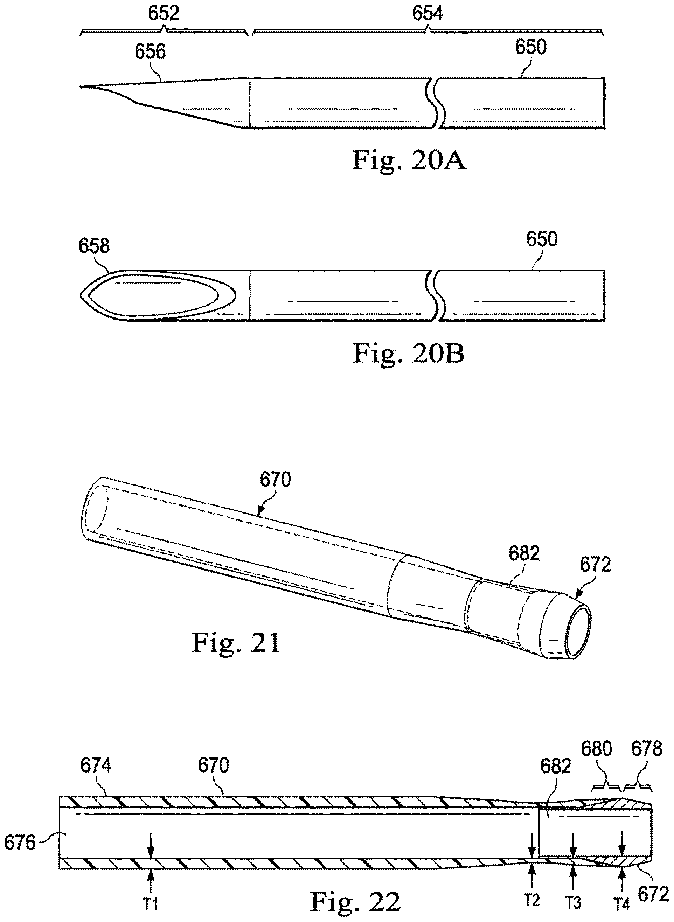

FIG. 20a illustrates a side views of a rigid distal portion 650 (e.g., rigid portion 309) of a biopsy tool. The rigid distal portion 650 includes a distal cutting section 652 and a shaft portion 654. The shaft portion 654 has a circular cross section and the cutting section 652 has an oblong or oval cross section created by a flattened and angled wall 656. The cutting section 652 includes a cutting surface 658. The circular volume of the portion 654 provides a large volume lumen to store large quantities of tissue. The oval or oblong cross section of the cutting section 652 may retain a sharp point for puncturing tissue while minimizing damage to the inner lumen wall of a sheath (e.g., sheath 304) through which the distal portion 650 may be passed.

FIGS. 21 and 22 illustrate a needle sheath 670 (e.g., sheath 304) that includes an elongate tubular member 674 and guard member 672. The elongate tubular member 674 includes a wall defining a channel 676. The guard member 672 includes a head portion 678, a tapered portion 680 and a tubular body portion 682. The body portion 682 is sized to fit within the channel 676 and may be secured by, teeth on the body portion 682 or channel 676, interlocking teeth on both body portion and channel, friction, or/or adhesive. The tapered portion 680 may also fit within the channel 676. The head portion 678 may extend distally of the distal tip of the tubular member 674 . The head portion may be tapered and may have a rounded edge to prevent tissue injury. The tubular member 674 may be formed from a flexible material such as Pebax that may be mixed with a lubricious material to allow the sheath to slide easily within the catheter. The guard member 672 may be made of a more rigid material than the tubular member 674. Suitable material may include metal or hardened plastic that resists damage by the point of a biopsy needle. The tubular member or the guard member may include a radiopaque marker to guide movement of the biopsy needle in the patient anatomy.

The sheath 670 may be contoured with a distal dip to permit greater bending and navigability of the distal end of the sheath 670. In one embodiment, the tubular member 674 may have a proximal wall thickness T1, an intermediate wall thickness T2, and a distal wall thickness T3. The guard member 672 may have a maximum wall thickness T4. The wall thicknesses T1, T3, and T4 are greater than the wall thickness T2 to provide a narrowed or hourglass shape to the sheath. In an alternative embodiment, the wall thicknesses T1 and T4 are greater than the wall thickness T2, and the thickness T3 may be greater than, the same as or less than the wall thickness T2.

One or more elements in embodiments of the invention (e.g., the processing of signals received from the input controls and/or control of the flexible catheter) may be implemented in software to execute on a processor of a computer system, such as control system 112. When implemented in software, the elements of the embodiments of the invention are essentially the code segments to perform the necessary tasks. The program or code segments can be stored in a non-transitory machine-readable storage media, including any media that can store information including an optical medium, semiconductor medium, and magnetic medium. Machine-readable storage media examples include an electronic circuit; a semiconductor device, a semiconductor memory device, a read only memory (ROM), a flash memory, an erasable programmable read only memory (EPROM); a floppy diskette, a CD-ROM, an optical disk, a hard disk, or other storage device. The code segments may be downloaded via computer networks such as the Internet, Intranet, etc. As described herein, operations of accessing, detecting, initiating, registered, displaying, receiving, generating, determining, moving data points, segmenting, matching, etc. may be performed at least in part by the control system 112 or the processors thereof.

Note that the processes and displays presented may not inherently be related to any particular computer or other apparatus. The required structure for a variety of these systems will appear as elements in the claims. In addition, the embodiments of the invention are not described with reference to any particular programming language. It will be appreciated that a variety of programming languages may be used to implement the teachings of the invention as described herein.

While certain exemplary embodiments of the invention have been described and shown in the accompanying drawings, it is to be understood that such embodiments are merely illustrative of and not restrictive on the invention, and that the embodiments of the invention not be limited to the specific constructions and arrangements shown and described, since various other modifications may occur to those ordinarily skilled in the art. The scope of the invention is defined by the appended claims solely.

## Claims

1. A medical tool (300) comprising:
an elongated tubular section (310, 310A, 310B, 364) having a body wall including a plurality of slits (314, 350, 360, 602, 612, 622);
a rigid needle tip (309, 309A, 309B, 363, 650) coupled to a distal end of the elongated tubular section;
a flexible jacket (318) covering a portion of the elongated tubular section; and
an elongated flexible member (316) coupled to a proximal end of the elongated tubular section, wherein a distal portion of the elongated flexible member covers a proximal portion of the flexible jacket;
**characterized in that**
the flexible jacket extends into a first portion of slits of the plurality of slits and a proximal portion of the elongated flexible member extends into a second portion of slits of the plurality of slits, and wherein the second portion of slits is not covered by the flexible jacket.

2. The medical tool of claim 1 wherein the plurality of slits are arranged in a spiral pattern or are arranged perpendicular to a longitudinal axis through the elongated tubular section.

3. The medical tool of claim 1 wherein the rigid needle tip is integrally formed with the elongated tubular section such that a lumen extends through the elongated tubular section and the rigid needle tip.

4. The medical tool of one of claims 1 and 2 wherein the flexible jacket is formed of a heat shrink tubing or wherein the flexible jacket is a thermoplastic.

5. The medical tool of claim 1, wherein the rigid needle tip is shaped in a curve and a distal point of the rigid needle tip is aligned along a centerline of the medical tool.

6. The medical tool of claim 1 wherein the elongated tubular section is formed of stainless steel.

7. The medical tool of claim 1 wherein the elongated flexible member includes a plurality of external longitudinal ribs extending parallel to a longitudinal axis of the elongated flexible member.

8. The medical tool of claim 1 wherein the elongated flexible member includes a plurality of external projections extending perpendicular to a longitudinal axis of the elongated flexible member.

9. The medical tool of claim 1 further comprising a sheath (304, 670) including a sheath channel (334, 676), wherein the elongated flexible member is slideably received within the sheath channel.

10. The medical tool of claim 9 wherein a distal end of the sheath is a higher stiffness than a proximal portion of the sheath or wherein the distal end of the sheath is formed of a polymer embedded with a hardening material.

11. The medical tool of claim 9 wherein a rigid guard member (322, 672) extends around a distal surface of a sheath wall surrounding the sheath channel to provide stiffness.

12. The medical tool of claim 9 further comprising a handle (306, 500) wherein a proximal end of the elongated flexible member is coupled to the handle and a proximal end of the sheath is coupled to the handle.

13. The medical tool of claim 12, wherein the handle is configured to limit movement of the elongated flexible member at one of a first longitudinal position and a second longitudinal position within the sheath channel or wherein the handle includes an adjustable needle stopper to limit movement of the elongated flexible member to the first longitudinal position or the second longitudinal position.

14. The medical tool of claim 1 further comprising a stylet (320) formed of an elastic material and sized to extend through the elongated tubular section and the rigid needle tip to straighten the elongated tubular section.

## Patentansprüche

1. Medizinisches Werkzeug (300), das umfasst:
einen länglichen röhrenförmigen Abschnitt (310, 310A, 310B, 364) mit einer Körperwand, die eine Vielzahl von Schlitzen (314, 350, 360, 602, 612, 622) enthält;
eine starre Nadelspitze (309, 309A, 309B, 363, 650), die mit einem distalen Ende des länglichen röhrenförmigen Abschnitts gekoppelt ist;
einen flexiblen Mantel (318), der einen Teil des länglichen röhrenförmigen Abschnitts bedeckt; und
ein längliches flexibles Element (316), das mit einem proximalen Ende des länglichen röhrenförmigen Abschnitts gekoppelt ist, wobei ein distaler Teil des länglichen flexiblen Elements einen proximalen Teil des flexiblen Mantels bedeckt;
**dadurch gekennzeichnet, dass**
der flexible Mantel sich in einen ersten Abschnitt von Schlitzen der Vielzahl von Schlitzen erstreckt und ein proximaler Abschnitt des länglichen flexiblen Elements sich in einen zweiten Abschnitt von Schlitzen der Vielzahl von Schlitzen erstreckt, und wobei der zweite Abschnitt der Schlitze nicht von dem flexiblen Mantel bedeckt ist.

2. Medizinisches Werkzeug nach Anspruch 1, wobei die Vielzahl der Schlitze spiralförmig oder senkrecht zu einer Längsachse durch den länglichen röhrenförmigen Abschnitt ausgelegt ist.

3. Medizinisches Werkzeug nach Anspruch 1, wobei die starre Nadelspitze einstückig mit dem länglichen röhrenförmigen Abschnitt gebildet ist, so dass sich ein Lumen durch den länglichen röhrenförmigen Abschnitt und die starre Nadelspitze erstreckt.

4. Medizinisches Werkzeug nach einem der Ansprüche 1 und 2, wobei der flexible Mantel aus einem Schrumpfschlauch gebildet ist oder wobei der flexible Mantel aus einem thermoplastischen Kunststoff besteht.

5. Medizinisches Werkzeug nach Anspruch 1, wobei die starre Nadelspitze in einer Kurve geformt ist und ein distaler Punkt der starren Nadelspitze entlang einer Mittellinie des medizinischen Werkzeugs ausgerichtet ist.

6. Medizinisches Werkzeug nach Anspruch 1, wobei der längliche röhrenförmige Abschnitt aus rostfreiem Stahl gebildet ist.

7. Medizinisches Werkzeug nach Anspruch 1, wobei das längliche flexible Element eine Vielzahl von äußeren Längsrippen enthält, die sich parallel zu einer Längsachse des länglichen flexiblen Elements erstrecken.

8. Medizinisches Werkzeug nach Anspruch 1, wobei das längliche flexible Element eine Vielzahl von äußeren Vorsprüngen enthält, die sich senkrecht zu einer Längsachse des länglichen flexiblen Elements erstrecken.

9. Medizinisches Werkzeug nach Anspruch 1, das ferner eine Hülle (304, 670) umfasst, die einen Hüllenkanal (334, 676) enthält, wobei das längliche flexible Element gleitend in dem Hüllenkanal aufgenommen ist.

10. Medizinisches Werkzeug nach Anspruch 9, wobei ein distales Ende der Hülse eine höhere Steifigkeit aufweist als ein proximaler Abschnitt der Hülse oder wobei das distale Ende der Hülse aus einem Polymer gebildet ist, in das ein härtendes Material eingebettet ist.

11. Medizinisches Werkzeug nach Anspruch 9, wobei sich ein starres Schutzelement (322, 672) um eine distale Oberfläche einer den Hülsenkanal umgebenden Hülsenwand erstreckt, um Steifigkeit zu gewährleisten.

12. Medizinisches Werkzeug nach Anspruch 9, das ferner einen Griff (306, 500) umfasst, wobei ein proximales Ende des länglichen flexiblen Elements mit dem Griff gekoppelt ist und ein proximales Ende der Hülle mit dem Griff gekoppelt ist.

13. Medizinisches Werkzeug nach Anspruch 12, wobei der Griff dafür konfiguriert ist, die Bewegung des länglichen flexiblen Elements in einer ersten Längsposition oder einer zweiten Längsposition innerhalb des Hüllenkanals zu begrenzen oder wobei der Griff einen einstellbaren Nadelstopper enthält, um die Bewegung des länglichen flexiblen Elements in die erste Längsposition oder die zweite Längsposition zu begrenzen.

14. Medizinisches Werkzeug nach Anspruch 1, das ferner ein Stilett (320) umfasst, das aus einem elastischen Material besteht und so bemessen ist, dass es sich durch den länglichen röhrenförmigen Abschnitt und die starre Nadelspitze erstreckt, um den länglichen röhrenförmigen Abschnitt zu begradigen.

## Revendications

1. Outil médical (300) comprenant :
une section tubulaire allongée (310, 310A, 310B, 364) ayant une paroi de corps comprenant une pluralité de fentes (314, 350, 360, 602, 612, 622) ;
une pointe d'aiguille rigide (309, 309A, 309B, 363, 650) couplée à une extrémité distale de la section tubulaire allongée ;
une enveloppe flexible (318) recouvrant une portion de la section tubulaire allongée ; et
un élément flexible allongé (316) couplé à une extrémité proximale de la section tubulaire allongée, une portion distale de l'élément flexible allongé recouvrant une portion proximale de l'enveloppe flexible ;
**caractérisé en ce que**
l'enveloppe flexible s'étend dans une première quantité de fentes de la pluralité de fentes et une portion proximale de l'élément flexible allongé s'étend dans une deuxième quantité de fentes de la pluralité de fentes, ladite deuxième quantité de fentes n'étant pas recouverte par l'enveloppe flexible.

2. Outil médical selon la revendication 1, dans lequel la pluralité de fentes est agencée selon un motif en spirale ou est agencée perpendiculairement à l'axe longitudinal traversant la section tubulaire allongée.

3. Outil médical selon la revendication 1, dans lequel la pointe d'aiguille rigide est formée d'un seul tenant avec la section tubulaire allongée, de telle sorte qu'une lumière s'étend à travers la section tubulaire allongée et la pointe d'aiguille rigide.

4. Outil médical selon l'une des revendications 1 et 2, dans lequel l'enveloppe flexible est formée d'un tube thermorétractable ou dans lequel l'enveloppe flexible est en matériau thermoplastique.

5. Outil médical selon la revendication 1, dans lequel la pointe d'aiguille rigide est de forme incurvée et un point distal de la pointe d'aiguille rigide est aligné sur une ligne médiane de l'outil médical.

6. Outil médical selon la revendication 1, dans lequel la section tubulaire allongée est réalisée en acier inoxydable.

7. Outil médical selon la revendication 1, dans lequel l'élément flexible allongé comprend une pluralité de nervures longitudinales extérieures s'étendant parallèlement à un axe longitudinal de l'élément flexible allongé.

8. Outil médical selon la revendication 1, dans lequel l'élément flexible allongé comprend une pluralité de saillies extérieures s'étendant perpendiculairement à un axe longitudinal de l'élément flexible allongé.

9. Outil médical selon la revendication 1, comprenant en outre une gaine (304, 670) comprenant un canal de gaine (334, 676) dans lequel l'élément flexible allongé est reçu de manière coulissante.

10. Outil médical selon la revendication 9, dans lequel l'extrémité distale de la gaine est plus rigide qu'une portion proximale de la gaine ou dans lequel l'extrémité distale de la gaine est réalisée dans un matériau polymère incrusté d'un matériau durcissant.

11. Outil médical selon la revendication 9, dans lequel un élément de protection rigide (322, 672) s'étend autour d'une surface distale d'une paroi de gaine entourant le canal de gaine pour assurer une rigidité.

12. Outil médical selon la revendication 9, comprenant en outre une poignée (306, 500), une extrémité proximale de l'élément flexible allongé étant couplée à la poignée et une extrémité proximale de la gaine étant couplée à la poignée.

13. Outil médical selon la revendication 12, dans lequel la poignée est conçue pour limiter le mouvement de l'élément flexible allongé au niveau d'une première position longitudinale ou d'une deuxième position longitudinale dans le canal de gaine, ou dans lequel la poignée comprend une butée d'aiguille réglable pour limiter le mouvement de l'élément flexible allongé vers la première position longitudinale ou la deuxième position longitudinale.

14. Outil médical selon la revendication 1, comprenant en outre un stylet (320) réalisé dans un matériau élastique et dimensionné pour traverser la section tubulaire allongée et la pointe d'aiguille rigide afin de redresser la section tubulaire allongée.
